# EUROPEAN PATENT APPLICATION

(11) **EP 0 597 693 A1**
(43) Date of publication of application: **18.05.1994**
(21) Application number: 93308977.3
(22) Date of filing: 10.11.1993
(51) Int. Cl.: B01J 21/10, B01J 37/04, C07C 45/74

(54) **Aldol condensation dehydration and catalyst therefor**

(30) Priority: 10.11.1992 JP 299616/92; 25.12.1992 JP 346498/92
(71) Applicant: SUMITOMO CHEMICAL COMPANY, LIMITED, Osaka (JP)
(72) Inventor: Fukao, Masami, Kurita-gun, Shiga 520-30 (JP); Sasaki, Kazuaki, Ibaraki-shi, Osaka 567 (JP); Suzukamo, Gohfu, Suita-shi, Osaka 564 (JP); Ishino, Masaru, Ichihara-shi, Chiba 299-01 (JP); Sasaki, Masao, Ibaraki-shi, Osaka 567 (JP)
(74) Representative: Cresswell, Thomas Anthony

(57) **Abstract**

The present invention provides an aldol condensation dehydration catalyst having high performance and giving condensation dehydrates at a high efficiency which is obtained by heating a mixture of an aqueous medium, magnesium oxide having an average particle size of 100µm or smaller and aluminum oxide and/or aluminum hydroxide having an average particle size of 100 µm or smaller at 350-600°C. The present invention further provides a process for producing the catalyst and a process for producing aldol condensation dehydrates.

## Description

The present invention relates to an aldol condensation dehydration catalyst, a process for producing the same and a process for preparing aldol condensation dehydration products using the same.

Catalysts for preparing aldol condensation hydration products by condensation dehydration of ketones or aldehydes, namely, aldol condensation dehydration catalysts include, for example, ① supported type catalysts prepared by supporting a calcium salt on alumina and heating it at 300-600°C (USP 4,535,187), ② coprecipitation type catalysts prepared by heating at 300-600°C a coprecipitate obtained by allowing an alkali to react with a solution of a magnesium salt and an aluminum salt (USP 4,086,188, USP 4,458,026 and USP 4,476,324), and ③ catalysts prepared by allowing pseudo-boehmite to react with a water-soluble acid such as nitric acid or acetic acid to produce once a gel, then allowing the gel to react with magnesium oxide or magnesium hydroxide and heating the resulting solid at 200 - 500°C (WO 90/12645).

However, preparation of the supported type catalysts ① requires repetition of impregnation and drying until calcium reaches a given concentration and the process for preparation is troublesome. For preparation of the coprecipitation type catalysts ②, a coprecipitate must be once produced and besides, the precipitate is filtrated with difficulty. Moreover, in the case of the catalysts ③, a gel must be once produced and in addition, the reaction product of the gel with magnesium compounds is difficult to filtrate. Thus, these are all unsatisfactory as industrial catalysts.

As a result of intensive research in an attempt to find superior aldol condensation dehydration catalysts, the inventors have found that mixture type catalysts obtained by a simple operation of mixing magnesium oxide having a specific particle size and aluminum oxide and/or aluminum hydroxide having a specific particle size with an aqueous medium unexpectedly have a high activity as aldol condensation dehydration catalysts. They have further made various investigations and accomplished the present invention.

That is, the present invention provides an aldol condensation dehydration catalyst prepared by heating at 350-600°C a mixture of an aqueous medium with magnesium oxide and aluminum oxide and/or aluminum hydroxide which respectively have an average particle size of 100 µ m or smaller.

The present invention further provides an industrially excellent process for producing aldol condensation dehydration catalysts which comprises heating at 350-600°C a mixture of an aqueous medium with magnesium oxide and aluminum oxide and/or aluminum hydroxide which respectively have an average particle size of 100 µ m or smaller.

The present invention further provides an industrially excellent process for preparing aldol condensation dehydration products by condensation dehydration of ketones and/or aldehydes using catalysts obtained by heating at 350-600°C a mixture of an aqueous medium with magnesium oxide and aluminum oxide and/or aluminum hydroxide which respectively have an average particle size of 100µ m or smaller.

The present invention is explained in more detail below.

The magnesium oxide used in the present invention may be either caustic-calcined magnesia or dead-burned magnesia, but the former is preferred because of its larger surface area.

The aluminum oxide and aluminum hydroxide used in the present invention include, for example, α - alumina, intermediate aluminas such as γ-, ρ-, χ- and η-aluminas, alumina hydrates such as boehmite, bialite and gibbsite, hydraulic alumina, amorphous alumina gel and alumina sol.

These magnesium oxide, aluminum oxide and aluminum hydroxide used have an average particle size of 100µ m or smaller, preferably 50 µ m or smaller. When those of larger than 100 µ m are used as they are, catalyst activity and selectivity lower. Therefore, they are ground to 100µ m or smaller.

When they are mixed in the presence of an aqueous medium, those which have an average particle size of 100 µ m or smaller may be directly mixed in the presence of an aqueous medium or may be previously mixed and further mixed in the presence of an aqueous medium.

When the average particle size is larger than 100 µ m, they may be ground to 100µ m or smaller in the presence of an aqueous medium and mixed or may be previously ground to 100µ m or smaller and mixed and then, further mixed in the presence of an aqueous medium.

Pulverizers such as ball mill and micronizers such as jet mill are normally used for grinding and mixing.

Ratio of magnesium oxide and aluminum oxide and/or aluminum hydroxide is usually 1:2 - 20:1, preferably 1:1 - 7:1 in terms of atomic ratio of magnesium and aluminum. The aqueous media include, for example, water, hydrous alcohols such as hydrous methanol, hydrous ethanol and hydrous ethylene glycol, hydrous ketones such as hydrous acetone, and hydrous ethers such as hydrous dioxane. Amount of the aqueous media is usually about 1 - 100 times the total weight of magnesium oxide and aluminum oxide and/or aluminum hydroxide.

Mixing temperature in the presence of aqueous medium is lower than the boiling point of the aqueous medium, usually 10 - 90°C. Mixing time is usually about 30 minutes - about 24 hours. The mixing may be effected in the air or in an inert gas atmosphere such as nitrogen gas.

The mixture containing aqueous medium may be dried as it is or after the aqueous medium has been filtered off.

The dried mixture is heated at a temperature of about 350 - 600°C. If the mixture is heated at lower than about 350°C or higher than about 600°C, catalyst performance is deteriorated.

The heating time is usually about 0.1 - 10 hours though it depends on the heating temperature.

Thus, aldol condensation dehydration catalysts having a remarkably excellent effect are obtained.

The catalysts may be supported on carriers or diluted with diluents such as glass beads. Furthermore, binders such as alumina sol may be added to improve mechanical strength.

The aldol condensation dehydration is carried out batchwisely or continuously.

The catalysts bed may be used in a fixed bed or a fluidized bed system.

As starting materials for aldol condensation dehydration reaction, mention may be made of, for example, ketones such as acetone, methyl ethyl ketone, methyl isobutyl ketone, diethyl ketone, cyclopentanone and cyclohexanone and aldehydes such as acetaldehyde and butylaldehyde.

The condensation dehydration reaction can be effected by conventional processes. For example, when mesityl oxide and isophorone are produced using acetone, the reaction temperature is usually about 250 - 400°C, preferably 270 - 330°C. The pressure is usually from atmospheric pressure to about 5 kg/cm². The feeding rate (LHSV) of the reactants is usually about 0.1 - 10 h⁻¹, preferably 1 - 5 h⁻¹.

The catalysts of the present invention have a high performance as aldol condensation dehydration catalysts and give condensation dehydration products at a high efficiency. For example, condensation dehydration of acetone efficiently gives a trimer isophorone.

Furthermore, the catalysts of the present invention can be prepared by a simple operation of mixing magnesium oxide having a specific particle size with aluminum oxide and/or aluminum hydroxide having a specific particle size in the presence of an aqueous medium. This is industrially advantageous.

The following nonlimiting examples illustrate the present invention.

The average particle size is measured by a laser diffraction type particle size distribution measuring apparatus.

### Example 1

In a planetary ball mill (manufactured by Frizsche Japan Co.) were charged 5.52 g of magnesium oxide (caustic-calcined magnesia manufactured by Konoshima Chemical Co., Ltd., average particle size: 283 µ m or larger) and 5.0 g of aluminum oxide (γ - alumina AC-11 manufactured by Sumitomo Chemical Co., Ltd., average particle size: 96µ m) and then thereto was added 55 g of water, followed by grinding and mixing for 15 minutes. Average particle size of the resulting mixture was 0.9 µ m. The mixture was dried at 100°C under 30 mmHg, then molded to 24 - 48 mesh and heated at 400°C for 2.5 hours in the air to obtain Catalyst A.

### Example 2

15 g of magnesium oxide (Starmag U manufactured by Konoshima Chemical Co., Ltd., average particle size: 17µ m), 12.75 g of aluminum oxide (hydraulic γ -alumina BK-103 manufactured by Sumitomo Chemical Co., Ltd., average particle size: 29µ m) and 228 g of water were charged in a flask provided with a stirrer and stirred at 25°C for 4 hours at 100 rpm. The mixture was subjected to filtration, dried at 100°C overnight, molded to 8 - 20 mesh and then heated at 450°C for 3 hours to obtain Catalyst B.

### Example 3

93 g of aluminum nitrate nonahydrate was dissolved in 500 g of water and then, to the solution were added dropwise 29.8 g of sodium hydroxide and 200 g of water at 25°C, followed by stirring for 3 hours. The precipitated solid was filtrated, washed with 500 ml of water 4 times and then dispersed in 700 ml of water. The dispersed particles had an average particle size of 33µ m.

Then, thereto was added 15 g of the same magnesium oxide as used in Example 2, followed by stirring at 25°C for 4 hours at 100 rpm. The mixture was filtrated, dried at 200°C for 3 hours, then molded to 8-20 mesh and heated at 400°C for 3.5 hours in the air to obtain Catalyst C.

### Example 4

Catalyst D was obtained in the same manner as in Example 3 except that aluminum nitrate nonahydrate was used in an amount of 55.85 g, sodium hydroxide was used in an amount of 17.9 g and magnesium oxide was used in an amount of 30 g and the mixing was effected at 80°C.

### Comparative Example 1

The mixing was carried out in the same manner as in Example 1 except that a flask provided with a stirrer was used in place of the planetary ball mill and the stirring was carried out at 50 rpm for 15 minutes. The resulting mixture had an average particle size of 211 µ m.

Then, drying, molding and heating were effected in the same manner as in Example 1 to obtain Catalyst E.

### Comparative Example 2

Catalyst F was obtained in the same manner as in Example 2 except that 21.7 g of magnesium hydroxide (manufactured by Kyowa Chemical Co., Ltd., average particle size: 20µ m) was used in place of magnesium oxide.

### Comparative Example 3

93 g of aluminum nitrate nonahydrate was dissolved in 500 g of water and then, to the solution were added dropwise 29.8 g of sodium hydroxide and 200 g of water at 25°C, followed by stirring for 3 hours. The precipitated solid was filtrated, washed with 500 ml of water 4 times and then dispersed in 350 ml of water. The dispersed particles had an average particle size of 30µ m.

95.38 g of magnesium nitrate hexahydrate was dissolved in 500 g of water and then, to the solution were added dropwise 29.8 g of sodium hydroxide and 200 g of water at 25°C, followed by stirring for 3 hours. The precipitated solid was filtrated, washed with 500 ml of water 4 times and then dispersed in 350 ml of water. The dispersed particles had an average particle size of 8 µ m.

Then, under stirring, thereto was added the aluminum hydroxide suspension obtained above, followed by stirring at 25 - 30°C for 3 hours. The mixture was filtrated and treated in the same manner as in Example 3 to obtain Catalyst G.

### Examples 5-8 and Comparative Examples 4-6

Each of the catalysts obtained in Examples 1-4 and Comparative Examples 1-3 was charged in a stainless steel reaction tube (inner diameter 16.5 mm ⌀ × 600 mm) having an outer diameter of 3/4 inch and after the reaction tube was heated to 290°C, acetone (purity 99.7%, water 0.3%) was fed thereto at an LHSV of 1 - 4 h⁻¹ with keeping the inner pressure at 2.8 kg/cm²G. Results of the reaction are shown in Table 1.

**Table 1**

| Example | Catalyst | LHSV | Composition of reaction mixture (wt%) | | |
|---|---|---|---|---|---|
| | | | Acetone | Mesityl oxide | Isophorone |
| Example 5 | A | 1 h⁻¹ | 58.5 | 2.4 | 20.7 |
| | | 2 | 67.1 | 3.3 | 16.4 |
| | | 3 | 72.5 | 3.6 | 13.5 |
| | | 4 | 76.7 | 3.7 | 11.4 |
| Example 6 | B | 1 | 70.6 | 3.7 | 11.8 |
| | | 2 | 76.4 | 4.5 | 9.5 |
| | | 3 | 81.3 | 4.5 | 7.7 |
| | | 4 | 84.8 | 4.3 | 6.3 |
| Example 7 | C | 1 | 55. | 2. | 21.3 |
| | | 2 | 63.9 | 2.9 | 17.7 |
| | | 3 | 69.9 | 3.3 | 14.8 |
| | | 4 | 74.7 | 3.4 | 12.3 |
| Example 8 | D | 1 | 67.6 | 3.3 | 11.8 |
| | | 2 | 78.1 | 3.8 | 8.6 |
| | | 3 | 82.8 | 3.6 | 6.7 |
| | | 4 | 85.9 | 3.3 | 5.3 |
| Comparative Example 4 | E | 1 | 83.8 | 6.2 | 3.8 |
| | | 2 | 88.5 | 5.3 | 2.2 |
| | | 3 | 90.8 | 4.7 | 1.5 |
| | | 4 | 92.3 | 4.1 | 1.2 |
| Comparative Example 5 | F | 1 | 85.3 | 6.1 | 3.3 |
| | | 2 | 88.3 | 5.5 | 2.2 |
| | | 3 | 90.9 | 4.8 | 1.5 |
| | | 4 | 92.9 | 4. | 1. |
| Comparative Example 6 | G | 1 | 79.6 | 5.7 | 6. |
| | | 2 | 85.1 | 5.4 | 4.1 |
| | | 3 | 88. | 4.6 | 3.3 |
| | | 4 | 89.7 | 4.3 | 2.6 |

Mesityl oxide: 4-methyl-3-penten-2-one + 4-methyl-4-penten-2-one
Isophorone: 3,5,5-trimethyl-2-cyclohexen-1-one + 3,5,5-trimethyl-3-cyclohexen-1-one

## Claims

1. A catalyst obtainable by heating at 350 to 600°C a mixture of an aqueous medium, magnesium oxide and at least one aluminium compound having an average particle size of 100µm or smaller and selected from aluminium oxide and aluminium hydroxide.

2. A catalyst according to claim 1, wherein the ratio of magnesium oxide to the total of aluminium compound or compounds is 1:2 to 20:1 in terms of the atomic ratio of magnesium to aluminium.

3. A catalyst according to claim 2, wherein the ratio of magnesium oxide to the total of aluminium compound or compounds is 1:1 to 7:1 in terms of the atomic ratio of magnesium to aluminium.

4. A catalyst according to any one of the preceding claims, wherein the magnesium oxide and the aluminium compound or compounds have an average particle size of 50µm or smaller.

5. A catalyst according to any one of the preceding claims, wherein the magnesium oxide is in the form of caustic-calcined magnesia.

6. A catalyst according to any one of the preceding claims, wherein the aluminium compound or compounds are in the form of a-alumina, an intermediate alumina, an alumina hydrate, a hydraulic alumina, an amorphous alumina gel or an alumina sol.

7. A catalyst according to any one of the preceding claims, wherein the aqueous medium is water, a hydrous alcohol, a hydrous ketone or a hydrous ether.

8. A process for producing a catalyst which comprises heating at 350 to 600°C a mixture of an aqueous medium, magnesium oxide having an average particle size of 100µm or smaller and at least one aluminium compound having an average particle size of 100µm or smaller and selected from aluminium oxide and aluminium hydroxide.

9. A process according to claim 8 for producing a catalyst according to any one of claims 2 to 7.

10. A process for preparing an aldol condensation dehydration product by condensation dehydration of at least one of a ketone and an aldehyde in the presence of a catalyst wherein the catalyst is one according to any one of claims 1 to 7.

11. A process according to claim 10 wherein a ketone is present and is acetone, methyl ethyl ketone, methyl isobutyl ketone, diethyl ketone, cyclopentanone or cyclohexanone.

12. A process according to claim 11, wherein the ketone is acetone.

13. A process according to claim 10 wherein an aldehyde is present and is acetaldehyde or butaldehyde.

14. A process according to any one of claims 10 to 13 wherein the reaction temperature is 250 to 400°C, the pressure is from about atmospheric pressure to 5 kg/cm², and feeding rate (Liquid hourly space velocity (LHSV)) of reactants is about 0.1 to 10 h⁻¹.
